(19) Europäisches Patentamt
European Patent Office
Office européen des brevets

(11) Veröffentlichungsnummer: **0 022 073**
**B1**

(12) **EUROPÄISCHE PATENTSCHRIFT**

(45) Veröffentlichungstag der Patentschrift: **30.01.85**

(51) Int. Cl.⁴: **C 07 D 303/24,** C 08 G 59/04

(21) Anmeldenummer: **80810192.7**

(22) Anmeldetag: **11.06.80**

# (54) Diglycidyläther von di-sekundären Alkoholen, ihre Herstellung und Verwendung.

(30) Priorität: **15.06.79 GB 7920990**

(43) Veröffentlichungstag der Anmeldung:
**07.01.81 Patentblatt 81/01**

(45) Bekanntmachung des Hinweises auf die Patenterteilung:
**30.01.85 Patentblatt 85/05**

(84) Benannte Vertragsstaaten:
**BE CH DE FR GB IT LI NL**

(56) Entgegenhaltungen:
**DE-A-2 057 848**
**DE-A-2 214 581**
**DE-A-2 223 645**
**DE-A-2 838 841**
**GB-A- 909 567**
**J. Chem. Soc. 1965, 2968-75**

(73) Patentinhaber: **CIBA-GEIGY AG**
**Postfach**
**CH-4002 Basel (CH)**

(72) Erfinder: **Bagga, Madan Mohan, Dr.**
**56, Hurrell Road**
**Cambridge (GB)**

**EP 0 022 073 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschriebung**

Die vorliegende Erfindung betrifft neue Diglycidyläther, Verfahren zu ihrer Herstellung und ihre Verwendung in härtbaren Mischungen.

In der GB—PS 909,567 wird die Bildung eines Monoäthers eines zweiwertigen Phenols durch Umsetzung von 1 Mol des zweiwertigen Phenols mit einem geeigneten Monoepoxid beschrieben. Unter Verwendung von Glycidol erhält man ein Mono-(2,3-dihydroxypropyl)-äther, während mit Phenylglycidyläther ein Mono-(2-hydroxy-2-phenoxypropyl)-äther erhalten wird. Die Umsetzung von 1 Mol eines solchen Monoäthers eines zweiwertigen Phenols mit 1 Mol Epichlorhydrin in Gegenwart eines geeigneten Kondensationskatalysators, zum Beispiel Bortrifluoridkomplex, führt hauptsächlich zur Bildung des Monochlorhydrinäthers, während die phenolische Hydroxylgruppe grösstenteils unreagiert bleibt. So wird zum Beispiel die Umsetzung des Phenylglycidyläthers mit einem zweiwertigen Phenol HO—X—OH und die anschliessende Behandlung mit Epichlorhydrin wie folgt angegeben:

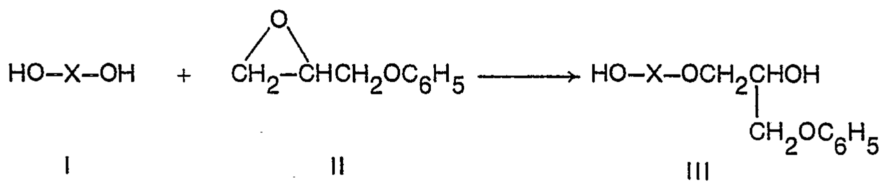

I II III

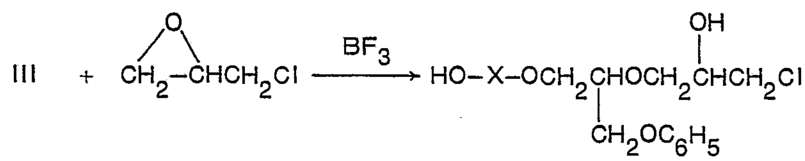


IV V

Solch ein Mono-(chlorhydrinäther) der Formel V wird dann bis zu äquimolaren Mengen mit einem Bis-(chlorhydrinäther) eines mehrwertigen Phenols gemischt und der Dehydrochlorierung unterworfen. Die erhaltenen Produkte, als hochmolekulare Produkte mit harzartigem Charakter beschrieben, stellen allgemein feste Produkte dar mit einem Schmelzpunkt von 43°C und höher. Bei Verwendung des Phenylglycidyläthers entsteht nach der Dehydrochlorierung neben anderen Verbindungen der Glycidyläther eines sekundären Monoalkohols nach folgender Weise:

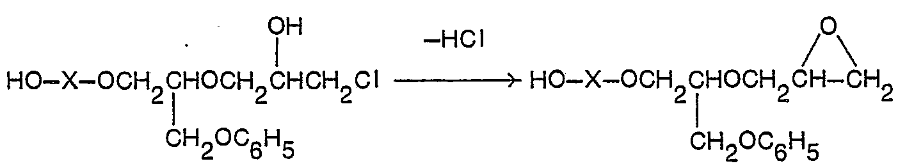


V VI

Dieses Produkt wird als brauchbar für Formassen beschrieben. In der GB—PS ist kein Hinweis enthalten, dass das zweiwertige Phenol der Formel I unter den angewendeten Bedingungen auch mit zwei Molen eines Monoepoxides reagieren kann unter Bildung des entsprechenden Bis-Adduktes der Formel VII

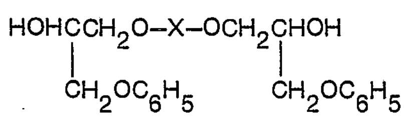

(VII),

wobei dieser Teil der Verbindung bei Behandlung mit Epichlorhydrin die Bis-(chlorhydrin)-äther bilden würde, woraus sich durch Dehydrochlorierung die entsprechenden Diglycidyläther herstellen liessen.

In den späteren GB—PSen 1,056,384 und 1,056,385 wird von den gleichen Erfindern wiederholt, dass die Umsetzung von 1 Mol eines zweiwertigen Phenols mit 1 Mol eines Monoepoxides zur Bildung eines eine monohydroxyaliphatische Aethergruppe enthaltenden zweiwertigen Phenols führt und dass bei der Umsetzung dieses Phenols mit Epichlorhydrin ausschliesslich die phenolische Hydroxylgruppe mit dem Epichlorhydrin reagiert, während die aliphatische Hydroxylgruppe unreagiert bleibt.

Wenn zum Beispiel das Reaktionsprodukt aus Phenylglycidyläther und Bisphenol A (Formel III) mit Epichlorhydrin behandelt wird, müsste demnach die Reaktion wie folgt ablaufen:

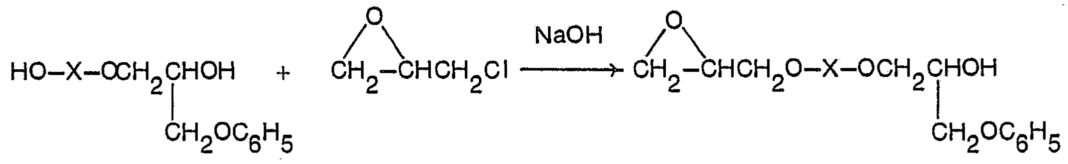


III IV VIII

In der GB—PS 1,512,814 werden Harze beschrieben, die wenigstens zwei photopolymerisierbare Gruppen und wenigstens zwei Epoxidgruppen enthalten, wobei eine oder mehrere Epoxidgruppen in der 2-(Glycidyloxy)propylen-Gruppierung enthalten sind. Diese Harze werden hergestellt, indem man die Hydroxylgruppen der 2-Hydroxypropylen-Gruppierung in bekannter Weise in Glycidyläthergruppen überführt. Diese Harze werden als brauchbar für Beschichtungen, insbesondere zur Herstellung von gedruckten Schaltungen, beschrieben.

Es wurde nun gefunden, dass bestimmte neue Diglycidyläther von di-(hydroxyaliphatischen) Aethergruppen enthaltenden zweiwertigen Phenolen härtbare Harze darstellen, die sich durch eine niedrige Viskosität ausweichnen und insbesondere als Giessharze eignen. Die meist als Giessharze gebräuchlichen Epoxidharze haben eine Viskosität von 10 Pa.s oder grösser bei Raumtemperatur. Zur Erniedrigung der Viskosität können den Mischungen nicht-reaktive Plastifizierungsmittel, Lösungsmittel oder reaktive Verdünner, wie Monoepoxide, zugegeben werden, doch werden dadurch die mechanischen Eigenschaften der gehärteten Epoxidharze meistens verschlechtert.

Gegenstand der vorliegenden Erfindung sind somit neue Diglycidyläther der allgemeinen Formel IX

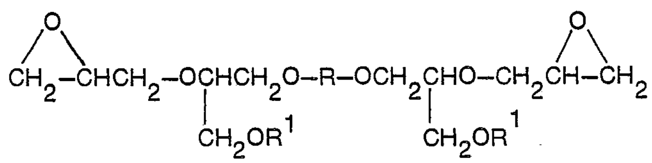
(IX),

worin R ein Phenylen, Naphthylen oder einen zwei oder drei Phenylene enthaltenden Rest bedeutet, wobei die Phenylene über eine einfache Bindung, —O—, —S—, —$SO_2$—, —SO—, —CO— oder ein Alkylen mit 1 bis 5 C-Atomen gebunden sind und jedes Phenylen oder Naphthylen gegebenenfalls am Ring durch ein oder zwei Alkyle mit 1 bis 6 C-Atomen oder durch 1 oder 2 Chlor- oder Bromatome substituiert sein kann, und jedes $R^1$ ein lineares oder verzweigtes, gegebenenfalls durch 1 bis 4 Chlor- oder Bromatome substituiertes Alkyl mit 1 bis 16 C-Atomen, ein lineares oder verzweigtes, gegebenenfalls durch 1 bis 4 Chlor- oder Bromatome substituiertes Alkenyl mit 2 bis 6 C-Atomen, ein gegebenenfalls am Ring durch ein oder zwei Chlor- oder Bromatome oder ein oder 2 Alkyle mit 1 bis 4 C-Atomen substituiertes Phenyl oder Naphthyl mit gesamthaft 6 bis 12 C-Atomen, ein gegebenenfalls am Ring durch ein oder zwei Chlor- oder Bromatome oder ein oder 2 Alkyle mit 1 bis 4 C-Atomen substituiertes Phenylalkyl oder Naphthylalkyl mit gesamthaft 7 bis 12 C-Atomen, ein einkerniges Cycloalkyl mit 3 bis 6 C-Atomen oder ein einkerniges Cycloalkylalkyl mit 4 bis 10 C-Atomen bedeutet.

Vorzugsweise haben beide $R^1$ die gleiche Bedeutung und stehen für ein Alkyl mit 1 bis 14 C-Atomen, Allyl, Cyclohexyl oder Benzyl.

Weitere bevorzugte Verbindungen der Formel IX sind solche, worin R ein m- oder p-Phenylen oder einen zwei Phenylene enthaltenden Rest bedeutet, wobei die beiden Phenylene in o,o'-, o,p'- oder p,p'-Stellung durch ein Alkylen mit 1 bis 4 C-Atomen miteinander verbunden sind.

Besonders bevorzugt sind solche Verbindungen der Formel IX, worin R einen Rest der Formel X

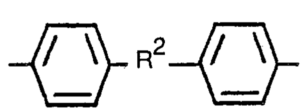
(X)

darstellt, worin $R^2$ für Methylen oder Isopropylen steht, und solche, worin jedes $R^1$ ein Alkyl mit 1 bis 12 C-Atomen, insbesondere 1 bis 6 C-Atomen, bedeutet.

Als spezifische Beispiele für die Diglycidyläther der Formel IX seien genannte:

2,2-Bis-(p-(3-butoxy-2-glycidyloxypropyloxy)-phenyl)-propan,
2,2-Bis-(p-(3-methoxy-2-glycidyloxypropyloxy)-phenyl)-propan,
2,2-Bis-(p-(3-äthoxy-2-glycidyloxypropyloxy)-phenyl)-propan,
2,2-Bis-(p-(3-dodecyloxy-2-glycidyloxypropyloxy)-phenyl)-propan,

2,2-Bis-(p-(3-tetradecyloxy-2-glycidyloxypropyloxy)-phenyl)-propan,
2,2-Bis-(p-(3-benzyloxy-2-glycidyloxypropyloxy)-phenyl)-propan,
Bis-(p-(3-butoxy-2-glycidyloxypropyloxy)-phenyl)-methan,
1,3-Bis-(3-phenoxy-2-glycidyloxypropyloxy)-benzol,
Bis-(p-(3-butoxy-2-glycidyloxypropyloxy)-phenyl)-sulfon,
2,2-Bis-(p-(3-cyclohexyloxy-2-glycidyloxypropyloxy)-phenyl)-propan,
2,2-Bis-(4-(3-butoxy-2-glycidyloxypropyloxy)-3,5-dibromophenyl)-propan,
2,2-Bis-(p-(3-allyloxy-2-glycidylpropyloxy)-phenyl)-propan, und
2,2-Bis-(p-(3-phenoxy-2-glycidyloxypropyloxy)-phenyl)-propan.

Die Diglycidyläther der Formel IX können aus den di-sekundären Alkoholen der Formel XI

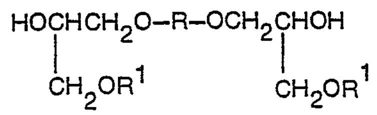
(XI) ,

worin R und $R^1$ die gleiche Bedeutung wie in Formel IX haben, hergestellt werden, indem man in bekannter Weise die alkoholischen Hydroxylgruppen durch Glycidyloxygruppen ersetzt.

Die Umwandlung eines Diols der Formel XI in einen Diglycidyläther der Formel IX wird durch Umsetzung mit Epichlorhydrin oder Glycerin-1,3-dichlorhydrin vorgenommen, wobei ein Bis-(chlorhydrin) der Formel XII

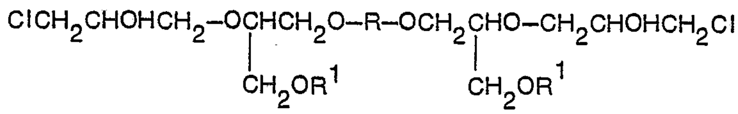
(XII)

entsteht, worin R und $R^1$ die gleiche Bedeutung wie in Formel IX haben. Das Bis-(chlorhydrin) der Formel XII wird dann dehydrochloriert, was vorzugsweise nach einer der folgenden Methoden durchgeführt wird:

A) In einem 2-Stufenverfahren wird zuerst das Diol der Formel XI mit wenigstens 1,5, vorzugsweise mit 1,6 bis 5, und insbesondere mit 1,8 bis 2,2 Aequivalenten Epichlorhydrin oder Glycerin-1,3-dichlor-hydrin in Gegenwart einer Lewissäure als Katalysator behandelt, wobei ein Bis-(chlorhydrin) der Formel XII entsteht. In der zweiten Stufe wird dann das Bis-(chlorhydrin) mit Alkali behandelt, wobei die Epoxidgruppen gebildet werden. Das Alkali ist normalerweise Na-hydroxid, doch können auch andere alkalische Substanzen, wie Ba-hydroxid oder K-carbonat für die Umwandlung der 1,2-Chlorhydringruppen in die 1,2-Epoxidgruppen verwendet werden.

B) Bei dem Einstufenverfahren wird ein Diol der Formel XI mit wenigstens 2,5, vorzugsweise 3 bis 8, Aequivalenten Epichlorhydrin in Gegenwart von Alkali, wie Na-hydroxid, und eines Phasenumwandlungskatalysators, wie Tetramethylammoniumchlorid, eines tertiären Amins oder einer quaternären Ammoniumbase behandelt. Wenigstens ein Teil des Ueberschusses an Epichlorhydrin wirkt als Chlorwasserstoffakzeptor und begünstigt die Bildung der Glycidyläther, wobei dieser Teil des Epichlorhydrins in Glycerin-1,3-dichlorhydrin umgewandelt wird.

Die Umsetzung kann nach beiden Methoden in einem Lösungsmittel, beispielsweise Kohlenwasserstoff, Aether oder Keton, durchgeführt werden, doch wird bei dem Einstufenverfahren ein Ueberschuss an Epichlorhydrin vorzugsweise als Lösungsmittel verwendet. Die Umsetzung wird allgemein bei erhöhter Temperatur, etwa 40—100°C, durchgeführt.

Die Diole der Formel XI sind aus der DE—OS 2,838,841 bekannt und können nach einem der folgenden Verfahren hergestellt werden:

1. Umsetzung von 1,5 bis 2,5, vorzugsweise etwa 1,8 bis 2,2 Mol eines Monoglycidyläthers der formel XIII

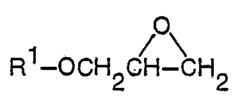
(XIII)

mit einem Mol eines zweiwertigen Phenols der Formel XIV

$$HO—R—OH \quad (XIV)$$

worin R und $R^1$ die gleiche Bedeutung wie in Formel IX haben.

Diese Umsetzung kann in Gegenwart eines basischen Katalysators, wie ein tertiäres Amin, eine quaternäre Ammoniumbase, ein Alkalimetallhydroxid oder ein quaternäres Ammoniumsalz vorgenommen werden und wird normalerweise durch Erhitzen der Ausgangsverbindungen auf 80 bis 180°C ohne Verwendung eines Lösungsmittels durchgeführt.

2. Umsetzung von wenigstens 2 Mol eines Alkohols der Formel XV

$$R^1OH \qquad (XV)$$

mit 1 Mol eines Diglycidyläthers eines zweiwertigen Phenols der Formel XVI

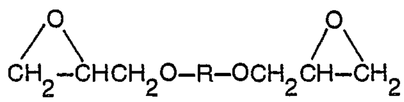

(XVI),

worin R und $R^1$ die gleiche Bedeutung wie in Formel IX haben.

Diese Umsetzung kann in Gegenwart eines basischen Katalysators, wie ein tertiäres Amin, eine quaternäre Ammoniumbase, ein Alkalimetallhydroxid oder ein quarternäres Ammoniumsalz bewirkt werden. Normalerweise erhitzt man die Ausgangsverbindung ohne Verwendung eines Lösungsmittels auf 80—180°C. Die Umsetzung kann auch in Gegenwart einer Lewissäure, wie Bortrifluoridkomplex oder Zinnchlorid, vorgenommen werden. Bei dieser Verfahrensweise wird gewöhnlich ein Ueberschuss des Alkohols der Formel XV angewendet und die Umsetzung zwischen 50 und 100°C durchgeführt. Der Ueberschuss an Alkohol wird dann vor der Glycidylierung durch Destillation entfernt.

3. Umsetzung von 1 Mol eines Alkalimetallsalzes von einem zweiwertigen Phenol der Formel XIV mit etwa 2 Mol eines Chlorhydrins der Formel XVII

$$R^1\text{—}OCH_2CHOHCH_2Cl \qquad (XVII)$$

worin $R^1$ die Bedeutung wie in Formel IX hat.

Die Ausgangsstoffe werden gewöhnlich auf eine Temperatur zwischen 50 und 150°C erhitzt, wobei vorzugsweise kein Lösungsmittel verwendet wird.

Die Diglycidyläther der Formel IX können mit den gebräuchlichen Härtungsmitteln für Epoxidharze gehärtet werden und ergeben unlösliche, unschmelzbare Stoffe mit technisch wertvollen Eigenschaften. Da die Diglycidyläther allgemein eine niedrige Viskosität aufweisen, werden sie insbesondere als Giessharze verwendet, doch können sie auch als Laminierharze, für Oberflächenbeschichtungen, als Tauchharze, in Pressmassen, als Isolierharze für die Elektroindustrie, als Dichtungsmassen oder als Klebstoffe Anwendung finden.

Gewünschtenfalls können die Diglycidylähter der Formel IX auch in Mischung mit anderen Epoxidharzen gehärtet werden.

Gegenstand der vorliegenden Erfindung sind somit auch härtbare Mischungen, enthaltend einen Diglycidyläther der Formel IX und ein Härtungsmittel für Epoxidharze sowie gegebenenfalls noch ein anderes Epoxidharz.

Als Beispiele für Härtungsmittel seien die gebräuchlichen Härtungsmittel für Epoxidharze genannt, einschliesslich der aliphatischen, cycloaliphatischen, aromatischen und heterocyclischen Amine, wie m- und p-Phenylendiamin, Bis-(4-aminophenyl)-methan, Anilin-Formaldehyd-Harz, Bis-(4-aminophenyl)-sulfon, Aethylendiamin, Propan-1,2-diamin, Propan-1,3-diamin, N,N-Diäthyläthylendiamin, Hexamethylendiamin, Diäthylentriamin, Triäthylentetramin, Tetraäthylenpentamin, N-(2-Hydroxyäthyl)-, N-(2-Hydroxypropyl)-, und N-(2-Cyanoäthyl)-diäthylentriamin, 2,2,4-Trimethylhexan-1,6-diamin, 2,3,3-Trimethylhexan-1,6-diamin, m-Xylylendiamin, N,N-Dimethyl- und N,N-Diäthylpropan-1,3-diamin, Aethanolamin, Bis-(4-aminocyclohexyl)-methan, 2,2-Bis-(4-aminocyclohexyl)-propan, 2,2-Bis-(4-amino-3-methylcyclohexyl)-propan, 3-Aminomethyl-3,5,5-trimethylcyclohexylamin (Isophorondiamin), und N-(2-Aminoäthyl)-piperazin; Dicyandiamid; Polyaminoamide, wie beispielsweise solche aus aliphatischen Polyaminen und dimerisierten oder trimerisierten Fettsäuren, aus Aminen mit einem stöchiometrischen Unterschuss an Polyepoxiden hergestellte Amin-Addukte; Isocyanate und Isothiocyanate; Polyphenole, wie Resorcin, Hydrochinon, 2,2-Bis-(4-hydroxyphenyl)-propan, Phenol-Aldehyd-Harze und ölmodifizierte Phenol-Aldehyd-Harze, Phosphorsäure, Polythiol, wie die im Handel unter der Bezeichnung "Thiokole" erhältlichen Polythiole, Polycarbonsäuren und ihre Anhydride, wie zum Beispiel Phthalsäureanhydrid, Tetrahydrophthalsäureanhydrid, Methylendomethylentetrahydrophthalsäureanhydrid, Nonenylbernsteinsäureanhydrid, Dodecenylbernsteinsäureanhydrid, Hexahydrophthalsäureanhydrid, Hexachlorendomethylentetrahydrophthalsäureanhydrid und Endomethylentetrahydrophthalsäureanhydrid und ihre Mischungen, Maleinsäureanhydrid, Bernsteinsäureanhydrid, Pyromellitsäureanhydrid, Benzophenon-3,3',4,4'-tetracarbonsäuredianhydrid, Polysebazinsäureanhydrid, Polyazelainsäureanhydrid, die Säure der zuvorgenannten Anhydride sowie auch Isophthalsäure, Terephthalsäure, Zitronensäure und Mellitsäure. Besonders bevorzugte Polycarbonsäuren oder Anhydride sind solche, die unterhalb von 60°C flüssig sind. Es können auch katalytisch wirkende Härtungsmittel verwendet werden, wie beispielsweise tertiäre Amine (zum Beispiel 2,4,6-Tris-(dimethylaminoäthyl)-phenol und andere Mannichbasen, N-Benzyldimethylamin und Triäthanolamin); Alkalimetallalkoxide von Alkoholen (zum Beispiel Na-Alkoholat von 2,4-Dihydroxy-3-hydroxymethylpentan), Zinnsalze von Alkansäuren (zum Beispiel Zinnoctanoat), Friedel-Crafts-Katalysatoren, wie Bortrifluorid und seine Komplexe und Chelate, die durch Umsetzung von Bortrifluorid mit zum Beispiel 1,3-Diketonen erhalten werden.

Mit den Härtungsmitteln können auch die geeigeneten Härtungs-beschleuniger eingesetzt werden. Bei Verwendung von Poly-(aminoamiden), Dicyandiamiden, Polythiole oder Polycarbonsäureanhydriden können Tertiäre Amine oder deren Salze, quaternäre Ammoniumverbindungen oder Alkalimetallalkoxide als Beschleuniger dienen. Beispiele von bestimmten Beschleuniger sind N-Benzyldimethylamin, 2,4,6-Tris-(dimethylaminomethyl)-phenyl, Imidazole und Triamylammoniumphenoxid.

Andere Beschleuniger, die ebenfalls eingesetzt werden können, sind insbesondere Magnesiumnitrat und Mangannitrat, fluorierte und chlorierte Carbonsäuren und deren Salze, wie Magnesiumtrifluoracetat, Na-trifluoracetat, Magnesiumtrichloracetat, Na-trichloracetat, Trifluormethansulfonsäure und ihre Salze, wie das Mangan, Zink-, Magnesium-, Nickel- und Cobaltsalz und Magnesiumperchlorat und Calziumperchlorat.

Die Menge des eingesetzten Härtungsmittels richtet sich nach der chemischen Natur des Härtungsmittels und nach den gewünschten Eigenschaften der härtbaren Mischung und des gehärteten Produktes. Die maximale Menge kann leicht ermittelt werden. Wenn das Härtungsmittel ein Amin ist, werden normalerweise 0,75 bis 1,25 Aequivalente Aminwasserstoff pro 1 Epoxidäquivalent eingesetzt. Wenn Polycarbonsäuren oder ihre Andydride eingesetzt werden, verwendet man gewöhnlich 0,4 bis 1,1 Aequivalente Carboxylgruppe bzw. Anhydridgruppe per 1 Aequivalent Epoxidgruppe. Bei der Verwendung von Polyphenolen als Härtungsmittel, setzt man 0,75 bis 1,25 phenolische Hydroxylgruppen per 1 Epoxidäquivalent ein. Katalytisch wirkende Härtungsmittel werden allgemein in Mengen von 1 bis 40 Gewichtsteilen pro 100 Gewichtsteile Epoxidharz eingesetzt.

Je nach Natur des verwendeten Härtungsmittels kann die Härtung bei Raumtemperatur oder bei höheren Temperaturen vorgenommen werden.

Die Härtung kann gewünschtenfalls auch in zwei Stufen vorgenommen werden, indem man zum Beispiel den Härtungsvorgang unterbricht oder, falls man ein Härtungsmittel für höhere Temperaturen einsetzt, die härtbare Mischung bei tieferen Temperaturen nur teilweise härten lässt. Die dabei erhaltenen Produkte sind noch schmelzbare und lösliche Präkondensate (sogenannte "B-Stufenharze") und eignen sich für Pressmassen, Sinterpulver oder Prepregs.

Von den Epoxidharen, welche in Mischung mit den Diglycidyläthern der Formel IX verwendet werden können, sind am besten solche mit terminierten Epoxidgruppen, zum Beispiel der Formel XVIII

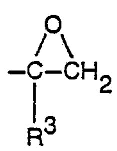

(XVIII),

worin $R^3$ ein Wasserstoffatom oder die Methylgruppe bedeutet, geeignet, und insbesondere solche Epoxidharze, worin die Glycidyl- oder $\beta$-Methylglycidylgruppen direkt an ein Sauerstoff-, Stickstoff- oder Schwefelatom gebunden sind. Solche Harze umfassen Polyglycidyl- und Poly-($\beta$-methylglycidyl)-ester, erhältlich durch Umsetzung von Polycarbonsäuren mit Epichlorhydrin, Glycerindichlorhydrin oder $\beta$-Methylepichlorhydrin, in Gegenwart von Alkali.

Die Polyglycidylester können sich von aliphatischen Carbonsäuren ableiten, wie zum Beispiel Oxalsäure, Bernsteinsäure oder Adipinsäure, Sebacinsäure, di- oder trimerisierter Linoleinsäure, von cycloaliphatischen Carbonsäuren, wie Hexahydrophthalsäure, 4-Methylhexahydrophthalsäure, Tetrahydrophthalsäure und 4-Methyltetrahydrophthalsäure, oder von aromatischen Carbonsäuren wie Phthalsäure, Isophthalsäure oder Terephthalsäure.

Andere Epoxidharze, die verwendet werden können, sind die Polyglycidyl- und Poly-($\beta$-methylglycidyl)-äther, die durch Umsetzung von mehr als eine alkoholische oder phenolische Hydroxylgruppe enthaltenden Verbindungen mit Epichlorhydrin, Glycerindichlorhydrin oder $\beta$-Methylepichlorhydrin unter alkalischen Bedingungen oder in Gegenwart eines sauren Katalysators mit anschliessender alkalischer Behandlung erhalten werden. Solche Polyglycidyläther können sich von aliphatischen Alkoholen, zum Beispiel Aethylenglykol und Poly-(oxyäthylen)-glykolen, wie Diäthylenglykol und Triäthylenglykol, Propylenglykol und Poly-(oxypropylen)-glykolen, Propan-1,3-diol, Butan-1,4-diol, Pentan-1,5-diol, Hexan-1,6-diol, Hexan-2,4,6-triol, Glycerin, 1,1,1-Trimethylolpropan und Pentaerythrit, von cycloaliphatischen Alkoholen, wie Chinit, 1,1-Bis-(hydroxymethyl)-cyclohex-3-en, Bis-(4-hydroxycyclohexyl)-methan und 2,2-Bis-(4-hydroxycyclohexyl)-propan oder von einen aromatischen Kern enthaltenden Alkoholen, wie N,N-Bis-(2-hydroxyäthyl)-anilin und 4,4'-Bis-(2-hydroxyäthylamino)-diphenylmethan ableiten. Bevorzugt sind solche Polyglycidyläther, die sich von Verbindungen ableiten, die zwei oder mehrere phenolische Hydroxylgruppen per Molekül aufweisen, wie Resorcin, Catechin, Hydrochinon, Bis-(4-hydroxyphenyl)-methan, 1,1,2,2-Tetrakis-(4-hydroxyphenyl)-äthan, 4,4'-Dihyroxy-diphenyl, Bis-(4-hydroxyphenyl)-sulfon, und insbesondere Phenol-Formaldehyd- oder Kresol-Formaldehyd-Novolakharze, 2,2-Bis-(4-hydroxyphenyl)-propan (Bisphenol A) und 2,2-Bis-(3,5-dibrom-4-hydroxyphenyl)-propan.

Ferner können Poly-(N-glycidyl)-Verbindungen verwendet werden, die durch Dehydrochlorierung von Umsetzungsprodukten aus Epichlorhydrin und Aminen mit mindestens zwei Aminwasserstoffatomen erhalten werden, wie Anilin, n-Butylamin, Bis-(4-aminophenyl)-methan, Bis-(4-aminophenyl)-sulfon, und Bis-(4-methylaminophenyl)-methan. Als weitere Poly-(N-glycidyl)-Verbindungen können Triglycidylisocyanurat, N,N'-Diglycidyl-Verbindungen von cyclischen Alkylenharnstoffen, wie Aethylenharnstoff und 1,3-Propylenharnstoff, und N,N'-Diglycidylverbindungen von Hydantoinen, wie 5,5-Dimethylhydantoin.

Epoxidharze, die durch Epoxidation von cyclischen und acryl-substituierten Polyolefinen erhalten werden, können ebenfalls eingesetzt werden, wie Vinylcyclohexandioxid, Limonendioxid, Dicyclopentadiendioxid, 3,4-Epoxydihydrodicyclopentadienylglycidyläther, der Bis-(3,4-epoxydihydrodicyclopentadienyl)-äther von Aethylenglykol, 3,4-Epoxycyclohexylmethyl-3',4'-epoxycyclohexancarboxylat und sein 6,6'-Dimethylderivat, Bis-(3,4-epoxycyclohexancarboxylat) von Aethylenglykol, das aus 3,4-Epoxycyclohexancarboxyaldehyd und 1,1-Bis-(hydroxymethyl)-3,4-epoxycyclohexan gebildete Acetal, Bis-(2,3-epoxycyclopentyl)-äther und epoxidiertes Butadien oder Copolymer aus Butadien mit äthylenischen Verbindungen, wie Styrol oder Vinylacetat.

Besonders geeingete Epoxidharze zum Vermischen mit den Diglycidyläthern der Formel IX sind Polyglycidyläther von 2,2-Bis-(4-hydroxyphenyl)-propan oder eines Novolaks aus Phenol, welches gegebenenfalls chlor- oder $C_1$—$C_4$-alkylsubstituiert ist, und Formaldehyd und welche einen Epoxidgehalt von mindestens 1,0 Aequivalenten/kg aufweisen.

Die erfindungsgemässen härtbaren Mischungen können ferner noch Plastifizierungsmittel, wie Dibutylphthalat, Diocylphthalat oder Trikresylphosphat, inerte Verdünnungsmittel oder sogenannte reaktive Verdünnungsmittel wie Diglycidylformaldehyd und insbesondere Monoepoxide, wie zum Beispiel Butylglycidyläther, Isooctylglycidyläther, Styroloxid, Glycidylacrylat, Glycidylmethacrylt und Glycidylester von synthetischen, hochverzeigten, vorwiegend tertiären, aliphatischen Monocarbonsäure, enthalten. Sie können ferner noch Additive enthalten, wie Füllstoffe, Verstärkungsmittel, Färbemittel, Fliessmittel, flammhemmende Stoffe und Formtrennmittel. Geeignete Streckmittel, Füllstoffe und Verstärkungsmittel umfassen Asbets, Asphalt, Bitumen, Glassfasern, Textilfasern, Kohlenstoff- oder Borfasern, Glimmer, Tonerde, Gips, Titandioxid, Kreide, Quarzmehl, Cellulose, Kaolin, gemahlener Dolomit, Wollastonit, Kieselerde mit grosser spezifischer Oberfläche (erhältlich unter dem Handelsnamen "Aerosil"), mit langkettigen Aminen modifizierte Tonerde (erhältlich unter dem Handelsnamen "Bentone"), gepulvertes Poly-(vinylchlorid); Polyolefin oder Aminoplast, Metallpulver, wie Aluminium- oder Eisenpulver. Flammschutzmittel, wie Antimontrioxid, können ebenfalls den härtbaren Mischungen zugegeben werden.

Die folgenden Beispiele beschreiben die Erfindung. Wenn nichts anderes vermerkt ist, bedeuten Teile Gewichtsteile. Der Epoxidgehalt wurde anhand einer Probe in Essigsäure und durch Titration mit einer Standardlösung aus Perchlorsäure in Essigssäure in Gegenwart von Tetraäthylammoniumbromid unter Verwendung von Kristallviolett als Indikator bestimmt.

Die in den Beispielen verwendeten Ausgangsverbindungen wurden wie folgt hergestellt.

2,2-Bis-(p-(3-butoxy-2-hydroxypropyloxy)-phenyl)-propan

*Methode I:* Bisphenol A (228 g; 1 Mol), Butylglycidyläther mit einem Epoxidgehalt von 7,4 Aequivalenten/kg (270,3 g; Aequivalente) und Benzyltrimethylammoniumchlorid (2,0 g) wurden verrührt und auf 110°C erhitzt, wobei eine exotherme Reaktion auftrat und die Temperatur auf 120°C stieg. Als die Temperatur zu fallenbegann wurde das Reaktionsgemisch erneut erhitzt und 1 Stunde bei 120°C und 3 Stunden bei 150°C gehalten. Dann wurde die Mischung abgekühlt und ein restlicher Epoxidgehalt von 0,11 Aequivalenten/kg bestimmt, was besagt, dass die Reaktion vollständig verlaufen ist.

*Methode II*: n-Butanol (1458 g; 19,7 Mol) wurde unter Rühren auf 60°C erhitzt. Bortrifluorid-diäthylätherkomplex (3,6 g) wurde zugegeben. Zu dieser Mischung werden 2,2-Bis-(p-glycidyloxyphenyl)-propan mit einem Epoxidgehalt von 5,73 Aequivalenten/kg (600 g; 3,44 Aequivalente) und n-Butanol (60 g; 0,81 Mol) während 5,5 Stunden zugetropft, wobei die Temperatur auf 60°C gehalten wurde. Danach wurde die Mischung noch 2 Stunden bei 60°C gerührt. Anschliessend wurde das überschüssige Butanol unter vermindertem Druck abdestilliert. Der Rückstand wog 839 g.

2,2-Bis-(p-3-äthoxy-2-hydroxypropyloxy)-phenyl)-propan

Dieses Produkt wurde nach Methode II hergestellt unter Verwendung der folgenden Ausgangsmaterialien: Methanol (1000 g; 21,7 Mol), Bortrifluorid-diäthylätherkomplex (2,4 g) und 2,2-Bis-(p-glycidyloxyphenyl)-propan mit einbem Epoxidgehalt von 5,3 Aequivalenten/kg (400 g; 2,12 Aequivalente) in Mischung mit Aethanol (80 g; 1,74 Mol). Der Rückstand wog 491 g.

2,2-Bis-(p-(3-methoxy-2-hydroxypropyloxy)-phenyl)-propan

Dieses Produkt wurde nach Methode II unter Verwendung folgender Ausgangsmaterialien hergestellt: Methanol (500 g; 15,6 Mol) Bortrifluorid-diäthylätherkomplex (1,2 g), und 2,2-Bis-(p-glycidyloxyphenyl)-propan mit einem Epoxidgehalt von 5,3 Aequivalenten/kg (200 g; 1,06 Aequivalente). Der Rückstand wog 232,6 g.

Bis-(p-3-butoxy-2-hydroxypropyloxy)-phenyl)-methan

Bis-(p-hydroxyphenyl)-methan (303 g; 1,5 Mol), Butylglycidyläther mit einem Epoxidgehalt von 7,45 Aequivalenten/kg (402,7 g; 6 Aequivalente) und Benzyltrimethylammoniumchlorid (3 g) werden miteinander verrührt und auf 100°C erhitzt, wobei exotherme Reaktion auftrat. Die Mischung wurde unter mässigem Kühlen auf 120°C gehalten und als die Temperatur zu fallen begann mehrmals für 1 Stunde auf 120°C erhitzt und anschliessend für 4 Stunden bei 150°C. Danach wurde das Reaktionsgemisch abgekühlt und der Epoxidgehalt zu 0,1 Aequivalenten/kg bestimmt, was besagt, das die Umsetzung vollständig verlaufen ist.

2,2-Bis-(p-(3-dodecyloxy- und -tetradecyloxy-2-hydroxypropyloxy)-phenyl)-propan

Eine im Handel erhältliche Mischung aus im wesentlichen linearen Dodecylglycidyläther und Tetradecylglycidyläther mit einem Epoxidgehalt von 3,4 Aequivalenten/kg (200 g; 284 Aequivalente), Bisphenol A (162,9 g; 0,71 Mol) und Benzyltrimethylammmoniumchlorid (1,4 g) wurden gemischt und dann auf 150°C erhitzt, wobei eine milde exotherme Reaktion auftrat und die Temperatur der Mischung während 30 Minuten auf 152°C stieg. Die Mischung wurde für weitere 30 Minuten bei dieser Temperatur belassen und dann während 4 Stunden auf 160°C erhitzt. Die Mischung hatte einen restlichen Epoxidgehalt von 0,15 Aequivalenten/kg, was besagt, dass die Umsetzung praktisch vollständig verlaufen ist.

Bis-(p-(3-butoxy-2-hydroxypropyloxy)-phenyl)-sulfon

4,4'-Dihydroxydiphenylsulfon (125,0 g; 0,5 Mol), Butylglycidyläther mit einem Epoxidgehalt von 7,45 (Aequivalenten/kg (134, 2 g; 1 Aequivalent), und Benzyltrimethylammoniumchlorid (1,0 g) wurden miteinander verrührt und während 1 Stunde auf 100°C, 2 Stunden auf 120°C und 4 Stunden auf 150°C erhitzt. Das Umsetzungsprodukt wurde dann abgekühlt und es wurde ein Epoxidgehalt von 0,03 Aequivalenten/kg ermittelt, womit die Reaktion im wesentlichen beendet war.

2,2-Bis-(p-(3-cyclohexyloxy-2-hydroxypropyloxy)-phenyl)-propan

Cyclohexanol (280 g; 2,8 Mol) wurde unter Rühren auf 60°C erhitzt. Bortrifluoriddiäthylätherkomplex (0,6 g) wurden zugegeben und zu dieser Mischung wurden 2,2-Bis-(p-glycidyloxyphenyl)-propan mit einem Epoxidgehalt von 5,73 Aequivalenten/kg (87,35 g; 0,5 Aequivalente) und Cyclohexanol (20 g; 0,2 Mol) während 1 Stunde zugetropft, wobei die Temperatur auf 60°C gehalten wurde. Danach wurde das Reaktionsgemisch für eine weitere Stunde bei 60°C gehalten und anschliessend das überschüssige Cyclohexanol unter vermindertem Druck abdestilliert. Der Rückstand wog 137,3 g.

2,2-Bis-(4-(3-butoxy-2-hydroxypropyloxy)-3,5-dibromphenyl)-propan

2,2-Bis-(3,5-dibrom-4-hydroxyphenyl)-propan (Tetrabrombisphenol A) (136 g; 0,25 Mol), Butylglycidyläther mit einem Epoxidgehalt von 7,45 Aequivalenten/kg (67,1 g; 0,5 Aequivalente) und Benzyltrimethylammoniumchlorid (0,5 g) wurden während 1 Stunde bei 120°C und 4,5 Stunden bei 150°C gerührt. Die Mischung hatte einen restlichen Epoxidgehalt von 0,01 Aequivalenten/kg, was besagt, dass die Umsetzung praktisch vollständig verlaufen ist.

2,2-Bis-(p-(3-allyloxy-2-hydroxypropyloxy)-phenyl)-propan

Bisphenol A (228 g; 1 Mol), Allylglycidyläther mit einem Epoxidgehalt von 8,64 Aequivalenten/kg (231,5 g; 2 Aequivalente) und Benzyltrimethylammoniumchlorid (2 g) wurden miteinander verrührt und auf 110°C erhitzt, bei welcher Temperatur eine exotherme Reaktion einsetzte und die Reaktionstemperatur auf 120°C brachte. Als die Temperatur zu fallen begann, wurde weiter erhitzt und das Reaktionsgemisch während 1,5 Stunden bei 120°C und 3 Stunden bei 150°C erhitzt. Das Reaktionsprodukt hatt einen restlichen Epoxidgehalt von 0,20 Aequivalenten/kg, was bedeutet, dass die Reaktion praktisch beendet war.

Analogs Diole können in gleicher Weise hergestellt werden:

2,2-Bis-(p-(3-benzyloxy-2-hydroxypropyloxy)-phenyl)-propan

2,2-Bis-(p-glycidyloxyphenyl)-propan (566,1 g) mit einem Epoxidgehalt von 5,2 Aequivalenten/kg, Benzylalkohol (324 g) und Tetramethylammoniumchlorid (1,5 g) wurden unter Stickstoff während 7 Stunden bei 180°C unter Rühren reagieren gelassen. Dann wurden weitere 0,5 g Tetramethylammoniumchlorid zugegeben und das Reaktionsgemisch für 13 Stunden unter Rühren auf 180°C erhitzt. Das Produkt hatt einen restlichen Epoxidgehalt von nur 0,5 Aequivalenten/kg.

1,3-Bis-(2-hydroxy-3-phenoxypropyloxy)-benzol

Resorcin (110 g), Phenylglycidyläther mit einem Epoxidgehalt von 6,08 Aequivalenten/kg (329,5 g) und 2-Phenylimidazol (0,4 g) wurden unter Rühren während 2 Stunden auf 130°C und danach während 3,5 Stundenauf 150°C erhitzt. Das erhaltene Produkt hatte einen restlichen Epoxidgehalt von 0,2 Aequivalenten/kg.

2,2-Bis-(p-(2-hydroxy-3-phenoxypropyloxy)-phenyl)-propan

*Methode I:* Phenylglycidyläther (156,6 g), Bisphenol A (114 g) und Benzyldimethylammoniumchlorid (2,7 g) wurden miteinander gemischt und vorsichtig auf 110°C erhitzt, bei welcher Temperatur eine exotherme Reaktion begann. Durch Kühlen wurde das Reaktionsgemisch auf 130°C gehalten. Nach Abklingen der exothermen Reaktion wurde die Mischung für 2 Stunden auf 130°C und dann für 3 Stunden auf 150°C erhitzt. Das erhaltene Produkt hatte einen Epoxidgehalt von nur 0,145 Aequivalenten/kg; das heisst, dass die Umsetzung praktisch vollständig verlaufen ist.

*Methode II:* Phenylglycidyläther (2243,6 g), Bisphenol A (1596 g) und 2-Phenylimidazol (0,5 g) wurden miteinander verrührt und vorsichtig auf 130°C erhitzt, bei welcher Temperatur eine milde exotherme Reaktion eintrat, die die Mischung während 40 Minuten auf 130—132°C hielt. Anschliessend wurde noch 1 Stunde bei 140°C und 6 Stunden bei 180°C erhitzt. Das erhaltene Produkt hatte einen Epoxidigehalt von nur 0,15 Aequivalenten/kg.

Beispiel 1

2,2-Bis-(p-(3-butoxy-2-hydroxypropyloxy)-phenyl)-propan (250 g; 1,0 Aequivalent), Epichlorhydrin (740 g; 8 Mol), und 50% wässriges Tetramethylammoniumchlorid (6,6 g) wurden verrührt und unter einem Teilvakuum erhitzt, sodass bei 55—58°C ein schonender Rückfluss eintrat. Eine 50% wässrige Na-hydroxidlösung (84 g; 105 Mol) wurde während 3 Stunden tropfenweise zugegeben, und das Wasser wurde kontinuierlich zusammen mit Epichlorhydrin als Azeotrop entfernt. Das Reaktionsgemisch wurde gekühlt und zur Entfernung des gebildeten Na-chlorids wiederholt mit Wasser gewaschen. Der Ueberschuss des Epichlorhydrins wurde durch Destillation unter vermindertem Druck entfernt und zurück blieb das 2,2-Bis-(p-(3-butoxy-2-glycidyloxypropyloxy)-phenyl)-propan (283 g), das einen Epoxidgehalt von 2,86 Aequivelenten/kg (theoretischer Wert: 3,28 Aequivalenten/kg) aufwies und eine Viskosität von 0,94 Pa.s hatte.

Beispiel 2

Beispiel 1 wurde wiederholt und als Ausgangsverbindungen 2,2-Bis-(p-(3-butoxy-2-hydroxypropyloxy)-phenyl)-propan (746,5 g; 3,0 Aequivalente), Epichlorhydrin (1110 g; 12 Mol), 50% wässriges Tetramethylammoniumchlorid (19,8 g), und 47% wässriges Na-hydroxid (268,5 g; 3,15 Mol) verwendet.

Die Ausbeute an 2,2-Bis-(p-3-butoxy-2-glycidyloxypropyloxy)-phenyl)-propan betrug 885,3 g, das einen Epoxidgehalt von 2,95 Aequivalenten/kg und eine Viskosität von 1,74 Pa.s bei 25°C aufwies.

Beispiel 3

Beispiel 1 wurde unter Verwendung von 3,3-Bis-(p-(3-äthoxy-2-hydroxypropyloxy)-phenyl)-propan (117,4 g; 0,5 Aequivalente), Epichlorhydrin (370 g; 4 Mol), 50% wässriges Tetramethylammoniumchlorid (3,3 g) und 47% wässriges Na-hydroxid (44,8 g; 0,525 Mol) wiederholt.

Die Ausbeute an 2,2-Bis-(p-(3-äthoxy-2-glycidyloxypropyloxy)-phenyl)-propan betrug 142,9 g. Das Produkt hatte einen Epoxidgehalt von 2,98 Aequivalenten/kg (theoretischer Wert: 3,44 Aequivalenten/kg) und eine Viskosität von 5,07 Pa.s bei 25°C.

Beispiel 4

Beispiel 1 wurde unter Verwendung von 2,2-Bis-(3-methoxy-2-hydroxypropyloxy)-phenyl)-propan (110,4 g; 0,5 Aequivalente), Epichlorhydrin (370 g; 4 Mol), 50% wässriges Tetramethylammoniumchlorid (3,3 g) und 47% wässriges Na-hydroxid (44,8 g; 0,525 Mol) wiederholt.

Die Ausbeute an 2,2-Bis-(p-(3-methoxy-2-glycidyloxypropoxy)-phenyl)-propan betrug 120 g. Dieses Produkt hatt einen Epoxidgehalt von 3,28 Aequivalenten/kg (theoretischer Wert: 3,6 Aequivalenten/kg) und eine Viskosität von 6,7 Pa.s bei 25°C.

Beispiel 5

Beispiel 1 wurde unter Verwendung von Bis-(*p*-(3-butoxy-2-hydroxypropyloxy)-phenyl)-methan (470,5 g; 2,04 Aequivalente), Epichlorhydrin (1480 g; 16 Mol), 50% wässriges Tetramethylammoniumchlorid (13,2 g) und 47% wässriges Na-hydroxid (179 g; 2,1 Mol) wiederholt.

Die Ausbeute an Bis-(p-(3-butoxy-2-glycidyloxypropyloxy)-phenyl)-methan betrug 526 g. Dieses Produkt hatte einen Epoxidgehalt von 2,88 Aequivalenten/kg (theoretischer Wert: 3,43 Aequivalenten/kg) und eine Viskosität von 0,85 Pa.s bei 25°C.

Nach dem gleichen Verfahrenkönnen 2,2-Bis-(*p*-(3-benzyloxy-3-glycidyloxypropyloxy)-phenyl)-propan, 1,3-Bis-(2-glycidyloxy-2-phenoxy-propyloxy)-benzol und 2,2-Bis-(p-(3-phenoxy-2-glycidyloxy-

propyloxy)-phenyl)-propan aus 2,2-Bis-(p-3-benzyloxy-2-hydroxypropyloxy)-phenyl)-propan, 1,3-Bis-(2-hydroxy-3-phenoxypropyloxy)-benzol bzw. 2,2-Bis-(p-(3-phenyloxy-2-hydroxypropyloxy)-phenyl)-propan hergestellt werden.

Beispiel 6

Beispiel 1 wurde unter Verwendung einer Mischung aus 2,2-Bis-(p-(3-dodecyloxy-2-hydroxypropyloxy)-phenyl)-propan und 2,2-Bis-(p-(3-tetradecyloxy-2-hydroxypropyloxy)-phenyl)-propan (408 g; 2 Aequivalente), Epichlorhydrin (740 g; 8 Mol), 50% wässriges Tetramethylammoniumchlorid (6,6 g) und 47% wässriges Na-hydroxid (89,5 g) wiederholt.

Die Ausbeute der Mischung aus 2,2-Bis-(p-(3-dodecyloxy-2-glycidyloxypropyloxy)-phenyl)-propan und 2,2-Bis-(p-(3-tetradecyloxy-2-glycidyloxypropyloxy)-phenyl)-propan betrug 386,5 g. Das erhaltene Produkt hatte einen Epoxidgehalt von 1,91 Aequivalenten/kg (theoretischer Wert: 2,15 Aequivalenten/kg) und eine Viskosität von 0,44 Pa.s bei 25°C.

Beispiel 7

Beispiel 1 wurde unter Verwendung von Bis-(p-(3-butoxy-2-hydroxypropyloxy)-phenyl)-sulfon (130 g; 0,5 Aequivalente), Epichlorhydrin (370 g; 4 Mol), 50% wässriges Tetramethylammoniumchlorid (3,3 g) und 47% wässriges Na-hydroxid (44,8 g; 0,525 Mol) wiederholt.

Die Ausbeute an Bis-(p-(3-butoxy-2-glycidyloxypropyloxy)-phenyl)-sulfon betrug 143 g. Das erhaltene Produkt hatte einen Epoxidgehalt von 2,86 Aequivalenten/kg (theoretischer Wert: 3,21 Aequivalente/kg) und eine Viskosität von 7,77 Pa.s bei 25°C.

Beispiel 8

Beispiel 1 wurde unter Verwendung von 2,2-Bis-(p-(3-cyclohexyloxy-2-hydroxypropyloxy)-phenyl)-propan (137,3 g; 0,5 Aequivalente), Epichlorhydrin (370 g; 4 Mol), 50% wässriges Tetramethylammoniumchlorid (3,3 g), und 47% wässriges Na-hydroxid (44,8 g; 0,525 Mol) wiederholt.

Die Ausbeute an 2,2-Bis-(p-(3-cyclohexyloxy-2-glycidyloxypropyloxy)-phenyl)-propan betrug 151 g. Das erhaltene Produkt war ein niederviskoses Harz mit einem Epoxidgehalt von 2,54 Aequivalenten/kg (theoretischer Wert: 3,07 Aequivalente/kg).

Beispiel 9

Beispiel 1 wurde unter Verwendung von 2,2-Bis-(4-(3-butoxy-2-hydroxypropyloxy)-3,5-dibromphenyl)-propan (203,1 g; 0,5 Aequivalente), Epichlorhydrin (370 g; 4 Mol), 50% wässriges Tetramethylammoniumchlorid (3,3 g), und 47% wässriges Na-hydroxid (44,8g; 0,525 Mol) wiederholt.

Die Ausbeute an 2,2-Bis-(p-(4-(3-butoxy-2-glycidyloxypropyloxy)-3,5-dibromphenyl)-propan betrug 221 g. Dieses Produkt hatte einen Epoxidgehalt von1,81 Aequivalenten/kg (theoretischer Wert: 2,16 Aequivalente/kg) und Viskosität von 63,0 Pa.s bei 25°C.

Beispiel 10

Beispiel 1 wurde unter Verwendung von 2,2-Bis-(p-(3-allyloxy-2-hydroxypropyloxy)-phenyl)-propan (461 g; 2 Aequivalente), Epichlorhydrin (1110 g; 12 Mol); 50% wässriges Tetramethylammoniumchlorid (13,2 g), und 47% wässriges Na-hydroxid (179 g; 2,1 Mol) wiederholt.

Die Ausbeute an 2,2-Bis-(p-(3-allyloxy-2-glycidyloxypropyloxy)-phenyl)-propan betrug 543 g. Das Produkt hatte einen Epoxidgehalt von 3,05 Aequivalenten/kg (theoretischer Wert: 3,52 Aequivalente/kg) und eine Viskosität von 1,3 Pa.s bei 25°C.

Beispiel 11

2,2-Bis-(p-3-butoxy-2-glycidyloxypropyloxy)-phenyl)-propan (100 g), hergestellt wie in Beispiel 1 beschrieben, wurden auf 60°C erhitzt. Dazu wurde 4,4'-Diaminodiphenylmethan (14,15 g) das vorher auf 100°C erhitzt wurde, zugegeben. Diese Mischung wurde in eine Form gegossen und während 4 Stunden auf 80°C und während 16 Stunden auf 140°C erhitzt. Der erhaltene Formkörper war durchsichtig, flexibel und hatte eine Glasumwandlungstemperatur von 20—25°C. Der Schermodul, gemessen mit einem Torsionspendel, betrug 1140 MPa bei —40°C, und 100 MPa bei 20°C. Die höchste Dehnfestigkeit, gemessen an vier Proben des gehärteten Harzes bei 23°C, betrug 11,6 ± 0,6 MPa und die Bruchdehnung betrug 122 ± 4%.

Beispiel 12

100 g des in Beispiel 1 hergestellten Diepoxides wurden mit 5,93 g Triäthylentetramin bei Raumtemperatur gemischt und in eine Form gegossen. Diese wurde bei Raumtemperatur über Nacht stehen gelassen und dann während 2 Stunden auf 80°C und 1 Stunde auf 100°C erhitzt. Der erhaltene Formkörper war durchsichtig und flexibel und hatte eine Glasumwandlungstemperatur von —7 bis —5°C. Sein Schermodul, gemessen mit einem Torsionspendel betrug 1130 MPa bei —40°C, 250 MPa bei —10°C und 0,7 MPa bei 20°C. Die höchste Dehnfestigkeit, gemessen an 3 Proben des gehärteten Harzes bei 22°C, betrug 0,46 ± 0,01 MPa und die Bruchdehnung war 36,3 ± 0,2%.

Beispiel 13

50 g des in Beispiel 1 hergestellten Diepoxides wurden auf 80°C erhitzt und 21,5 g auf ebenfalls bis 80°C erwärmtes Hexahydrophthalsäureanhydrid wurden zugegeben. Zu dieser Mischung wurde 1 g N-Benzyldimethylamin als Beschleuniger zugefügt. Nach dem Durchmischen wurde das Gemisch in eine Form gegossen und während 3 Stunden auf 80°C und 4 Stunden auf 120°C erhitzt. Der erhaltene Formkörper war durchsichtig und flexibel und hatte eine Glasumwandlungstemperatur von 30 bis 32°C. Die Scherfestigkeit des Formkörpers, gemessen mit einem Torsionspendel, betrug 1060 MPa bei −40°C, 1000 MPa bei −10°C und 750 MPa bei 20°C. Die höchste Dehnfestigkeit, gemessen an 5 Proben des gehärteten Harzes, betrug 23,9 ± 1,6 MPa und die Bruchdehnung war etwa 135%.

Beispiel 14

Je 25 g der in den Beispielen 3, 4 und 5 hergestellten Diepoxide wurden auf 60°C erhitzt und mit äquivalenten Mengen an 4,4'-Diaminodiphenylmethan (DDM) gemischt. Die Mischungen wurden in Formen gegossen und während 4 Stunden auf 80°C und 16 Stunden auf 140°C erhitzt.

Die Eigenschaften der gehärteten Formkörper sind in Tabelle 1 angegeben:

TABELLE 1

| Diepoxid gemäss Beispiel | Menge an DDM (g) | Aussehen des gehärteten Formkörpers | Glasumwandlungstemperatur (°C) |
|---|---|---|---|
| 3 | 3,8 | durchsichtig, flexibel | 55 |
| 4 | 4,17 | durchsichtig, flexibel | 65 |
| 5 | 3,67 | durchsichtig, flexibel | 26 |

Beispiel 15

25 g des in Beispiel 4 hergestellten Diepoxides wurden auf 80°C erhitzt und dann wurde 1 g Bortrifluoridäthylaminkomplex zugegeben. Die Mischung wurde solange gerührt, bis der Komplex sich vollständig in Harz aufgelöst hatte. Danach wurde das Harz in eine Form gegossen und während 3 Stunden bei 120°C und 3 Stunden bei 150°C erhitzt. Es wurde ein durchsichtiger, flexibler Formkörper mit einer Glasumwandlungstemperatur von 28°C erhalten.

**Patentansprüche**

1. Diglycidyläther der allgemeinen Formel IX

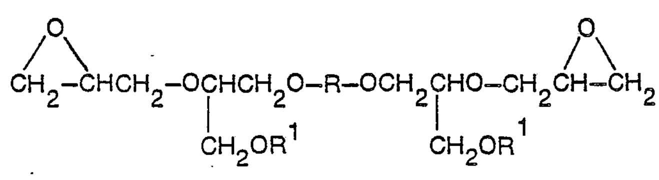

(IX),

worin R ein Phenylen, Naphthylen oder einen zwei oder drei Phenylene enthaltenden Rest bedeutet, wobei die Phenylene über eine einfache Bindung, —O—, —S—, —$SO_2$—, —SO—, —CO— oder ein Alkylen mit 1 bis 5 C-Atomen gebunden sind und jedes Phenylen oder Naphthylen gegebenenfalls am Ring durch ein oder zwei Alkyle mit 1 bis 6 C-Atomen oder durch 1 oder 2 Chlor- oder Bromatome substituiert sein kann, und jedes $R^1$ ein lineares oder verzweigtes, gegebenenfalls durch 1 bis 4 Chlor- oder Bromatome substituiertes Alkyl mit 1 bis 16 C-Atomen, ein lineares oder verzweigtes, gegebenenfalls durch 1 bis 4 Chlor- oder Bromatome substituiertes Alkenyl mit 2 bis 6 C-Atomen, ein gegebenenfalls am Ring durch ein oder zwei Chlor- oder Bromatome oder ein oder 2 Alkyle mit 1 bis 4 C-Atomen substituiertes Phenyl oder Naphthyl mit gesamthaft 6 bis 12 C-Atomen, ein gegebenenfalls am Ring durch ein oder zwei Chlor- oder Bromatome oder ein oder 2 Alkyle mit 1 bis 4 C-Atomen substituiertes Phenylalkyl oder Naphthylalkyl mit gesamthaft 7 bis 12 C-Atomen, ein einkerniges Cycloalkyl mit 3 bis 6 C-Atomen oder ein einkerniges Cycloalkylalkyl mit 4 bis 10 C-Atomen bedeutet.

2. Diglycidyläther gemäss Anspruch 1, worin in Formel IX beide $R^1$ die gleiche Bedeutung haben und je für ein Alkyl mit 1 bis 14 C-Atomen, Allyl, Cyclohexyl oder Benzyl stehen.

3. Diglycidyläther gemäss Anspruch 1 oder 2, worin R in Formel IX ein m- oder p-Phenylen oder einen zwei Phenylene enthaltenden Rest bedeutet, wobei die beiden Phenylene in o,o'-, o,p'- oder p,p'-Stellung durch ein Alkylen mit 1 bis 4 C-Atomen miteinander verbunden sind.

4. Diglycidyläther gemäss Anspruch 1, worin in Formel IX R einen Rest der Formel X

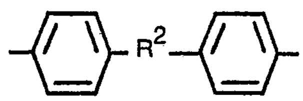

(X),

wobei $R^2$ für Methylen oder Isopropylen steht, und jedes $R^1$ ein Alkyl mit 1 bis 12 C-Atomen bedeuten.

5. Diglycidyläther gemäss Anspruch 1 mit folgenden Bezeichnunzen:
2,2-Bis-(p-(3-butoxy-2-glycidyloxypropyloxy)-phenyl)-propan,
2,2-Bis-(p-(3-methoxy-2-glycidyloxypropyloxy)-phenyl)-propan,
2,2-Bis-(p-(3-äthoxy-2-glycidyloxypropyloxy)-phenyl)-propan,
2,2-Bis-(p-(3-dodecyloxy-2-glycidyloxypropyloxy)-phenyl)-propan,
2,2-Bis-(p-(3-tetradecyloxy-2-glycidyloxypropyloxy)-phenyl)-propan,
2,2-Bis-(p-(3-benzyloxy-2-glycidyloxypropyloxy)-phenyl)-propan,
Bis-(p-(3-butoxy-2-glycidyloxypropyloxy)-phenyl)-methan,
1,3-Bis-(3-phenoxy-2-glycidyloxypropyloxy)-benzol,
Bis-(p-(3-butoxy-2-glycidyloxypropyloxy)-phenyl)-sulfon,
2,2-Bis-(p-(3-cyclohexyloxy-2-glycidyloxypropyloxy)-phenyl)-propan,
2,2-Bis-(4-(3-butoxy-2-glycidyloxypropyloxy)-3,5-dibromophenyl)-propan,
2,2-Bis-(p-(3-allyloxy-2-glycidyloxypropyloxy)-phenyl)-propan, und
2,2-Bis-(p-(3-phenoxy-2-glycidyloxypropyloxy)-phenyl)-propan.

6. Verfahren zur Herstellung von Diglycidyläthern der Formel IX gemäss Anspruch 1, dadurch gekennzeichnet, dass man einen disekundären Alkohol der Formel XI

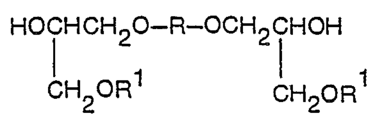

(XI)

worin R und $R^1$ die gleiche Bedeutung wie in Formel IX haben, mit Epichlorhydrin oder Glycerin-1,3-dichlorhydrin zu einem Bis-(chlorhydrin) der Formel XII umsetzt

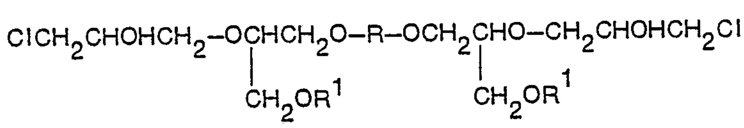

(XII)

worin R und $R^1$ die gleiche Bedeutung wie in Formel IX haben, und anschliessend das Bis-(chlorhydrin) der Formel XII in bekannter Wiese dehydrochloriert.

7. Verfahren gemäss Anspruch 6, worin der Alkohol der Formel XI mit wenigstens 1,5 Aequivalenten Epichlorhydrin oder Glycerin-1,3-dichlorhydrin in Gegenwart einer Lewissäure als Katalysator umgesetzt wird und das entsphrechende Bis-(chlorhydrin) der Formel XII dann mit einem Alkali behandelt wird.

8. Verfahren gemäss Anspruch 6, worin der Alkohol der Formel XI mit Epichlorhydrin oder Glycerin-1,3-dichlorhydrin im Temperaturbereich von 40—100°C behandelt wird.

9. Verfahren gemäss Anspruch 6, worin ein Alkohol der Formel XI verwendet wird, der durch Umsetztung von

a) 1,5 bis 2,5 Mol eines Monoglycidyläther der Formel XIII

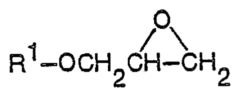 (XIII)

mit 1 Mol eines zweiwertigen Phenols der Formel XIV

$$HO—R—OH \qquad (XIV)$$

b) wenigstens 2 Mol eines Alkohols der Formel XV

$$R^1—OH \qquad (XV)$$

mit 1 Mol eines Diglycidyläthers eines zweiwertigen Phenols der Formel XVI

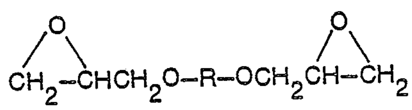 (XVI)

oder

c) 1 Mol eines Alkalimetallsalzes eines zweiwertigen Phenols der Formel XIV mit etwa 2 Mol eines Chlorhydrins der Formel XVII

$$R^1—OCH_2CHOHCH_2Cl \qquad (XVII)$$

worin R uns $R^1$ die gleiche Bedeutung wie in Anspruch 6 haben, erhalten wurde.

10. Härtbare Mischungen, enthaltend einen Diglycidyläther gemäss Anspruch 1 und ein Härtungsmittel für Epoxidharze.

**Claims**

1. A diglycidyl ether of the general formula IX

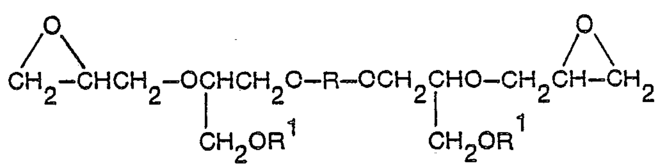 (IX),

wherein

R is a phenylene or naphthylene group, or a radical containing two or three phenylene groups, which phenylene groups are linked through a carbon-carbon bond, —O—, —S—, —$SO_2$—, —SO—, —CO— or a $C_1$—$C_5$ alkylene group, and each phenylene or naphthylene group may be substituted in the ring by one or two $C_1$—$C_6$ alkyl groups or by one or two chlorine or bromine atoms, and each $R^1$ is a linear or branched $C_1$—$C_{16}$ alkyl group which is unsubstituted or substituted by 1 to 4 chlorine or bromine atoms, or is a linear or branched $C_2$—$C_6$ alkenyl group which is unsubstituted or substituted by 1 to 4 chlorine or bromine atoms, or is a phenyl or naphthyl group which is unsubstituted or substituted in the ring by one or two chlorine or bromine atoms or by one or two $C_1$—$C_4$ alkyl groups and having in all 6 to 12 carbon atoms, or is a phenylalkyl or naphthylalkyl group which is unsubstituted or substituted in the ring by one or two chlorine or bromine atoms or by one or two $C_1$—$C_4$ alkyl groups and having in all 7 to 12 carbon atoms, or is a mononuclear $C_3$—$c_6$ cycloalkyl group or a mononuclear $C_4$—$C_{10}$ cycloalkylalkyl group.

2. A diglycidyl ether according to claim 1, wherein each $R^1$ in formula IX has the same meaning and each is a $C_1$—$C_{14}$ alkyl group, or is an allyl, cyclohexyl or benzyl group.

3. A diglycidyl ether according to either claim 1 or claim 2, wherein R in formula IX is an m- or p-phenylene group or is a radical containing two phenylene groups, both of which phenylenes are linked in the o,o′-, o,p′- or p,p′-position by a $C_1$—$c_4$ alkylene group.

4. A diglycidyl ether according to claim 1, wherein R in formula IX is a radical of formula X

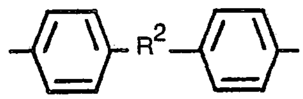 (X),

wherein

$R^2$ is a methylene or isopropylene group, and each $R^1$ is a $C_1$—$C_{12}$ alkyl group.

5. A diglycidyl ether according to claim 1, selected from the group consisting of:
2,2-bis(p-(3-butoxy-2-glycidyloxypropyloxy)phenyl)propane,
2,2-bis(p-(3-methoxy-2-glycidyloxypropyloxy)phenyl)propane,
2,2-bis(p-(3-ethoxy-2-glycidyloxypropyloxy)phenyl)propane,
2,2-bis(p-(3-dodecyloxy-2-glycidyloxypropyloxy)phenyl)propane,
2,2-bis(p-(3-tetradecyloxy-2-glycidyloxypropyloxy)phenyl)propane,
2,2-bis(p-(3-benzyloxy-2-glycidyloxypropyloxy)phenyl)propane,
bis-(p-3-butoxy-2-glycidyloxypropyloxy)phenyl)methane,
1,3-bis(3-phenoxy-2-glycidyloxypropyloxy)benzene,
bis(p-(3-butoxy-2-glycidyloxypropyloxy)phenyl)sulfone,
2,2-bis(p-(3-cyclohexyloxy-2-glycidyloxypropyloxy)phenyl)propane,
2,2-bis(4-(3-butoxy-2-glycidyloxypropyloxy)phenyl)-3,5-dibromo-phenyl)propane,
2,2-bis(p-(3-allyloxy-2-glycidyloxypropyloxy)phenyl)propane, and
2,2-bis(p-(3-phenoxy-2-glycidyloxypropyloxy)phenyl)propane.

6. A process for the preparation of a diglycidyl ether of formula IX according to claim 1, which comprises reacting a disecondary alcohol of formula XI

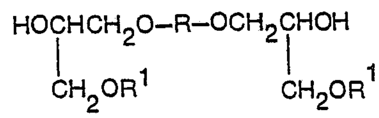 (XI)

wherein

R and $R^1$ are as defined in formula IX, with epichlorohydrin or glycerol-1,3-dichlorohydrin to form a bis(chlorohydrin) of formula XII

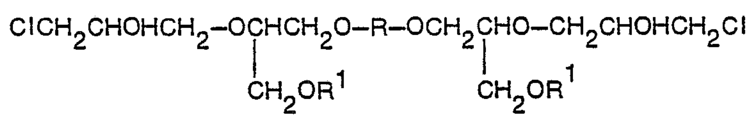 (XII)

wherein

R and $R^1$ are as defined in formula IX, and subsequently dehydrochlorinating the bis(chlorohydrin) of formula XII in known manner.

7. A process according to claim 6, wherein the alcohol of formula XI is reacted with at least 1.5 equivalents of epichlorohydrin or glycerol-1,3-dichlorohydrin in the presence of a Lewis acid as catalyst, and the resultant bis(chlorohydrin) of formula XII is subsequently treated with an alkali.

8. A process according to claim 6, wherein the alcohol of formula XI is treated with epichlorohydrin or glycerol-1,3-dichlorohydrin in the temperature range from 40—100°C.

9. A process according to claim 6, which comprises the use of an alcohol which has been obtained by reacting

a) 1.5 to 2.5 moles of a monoglycidyl ether of formula XIII

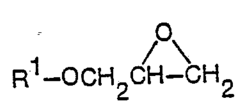 (XIII)

with 1 mole of a dihydric phenol of formula XIV

HO—R—OH (XIV)

b) at least 2 moles of an alcohol of formula XV

$R^1—OH$ (XV)

with 1 mole of a diglycidyl ether of a dihydric phenol of formula XVI

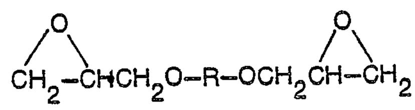

(XVI)

or

c) 1 mole of an alkali metal salt of a dihydric phenol of formula XIV with about 2 moles of a chlorohydrin of formula XVII

$R^1—OCH_2CHOHCH_2Cl$ (XVII)

wherein

R and $R^1$ are as defined in claim 6.

10. A curable mixture containing a diglycidyl ether according to claim 1 and a hardener for epoxy resins.

**Revendications**

1. Ethers diglycidyliques répondant à la formule générale IX:

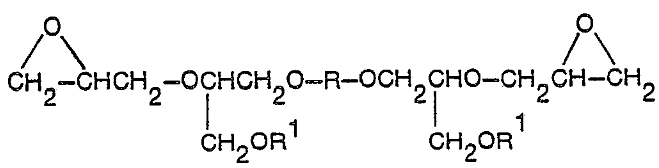

(IX)

dans laquelle,

R représente un phénylène, un naphtylène ou un radical contenant deux ou trois phénylènes, les phénylènes étant reliés par une liaison simple, —O—, —S—, —$SO_2$—, —SO—, —CO— ou un alkylène en $C_1$—$C_5$, et chaque phénylène ou naphtylène, pouvant porter sur son noyau un ou deux alkyles en $C_1$—$C_6$ ou un ou deux atomes de chlore ou de brome, et les

$R^1$ représentent chacun un alkyle en $C_1$—$C_{16}$, linéaire ou ramifié, éventuellement porteur d'un à quatre atomes de chlore ou de brome, un alcényle en $C_2$—$C_6$ linéaire ou ramifié, éventuellement porteur d'un à quatre atomes de chlore ou de brome, un radical phényle ou naphtyle dont le noyau porte éventuellement unou deux atomes de chlore ou de brome ou un ou deux alkyles en $C_1$—$C_4$ et qui contient au total de six à douze atomes de carbone, un radical phénylalkyle ou naphtylalkyle dont le noyau porte éventuellement un ou deux atomes de chlore ou de brome ou un ou deux alkyles en $C_1$—$C_4$ et qui contient au total de sept à douze atomes de carbone, un cycloalkyle à un seul noyau contenant de trois à six atomes de carbone ou un cycloalkyl-alkyle mononucléaire contenant de quatre à dix atomes de carbone.

2. Ethers diglycidyliques de formule IX selon la revendication 1 dans lesquels les deux symboles $R^1$ ont la même signification et représentent chacun un alkyle contenant de un à quatorze atomes de carbone, un allyle, un cyclohexyle ou un benzyle.

3. Ethers diglycidyliques de formule IX selon l'une des revendications 1 et 2, dans lesquels R représente un radical m-phénylène ou p-phénylène ou un radical contenant deux radicaux phénylènes reliés l'un à l'autre en o,o', o,p', ou p,p' par un alkylène contenant de 1 à 4 atomes de carbone.

4. Ethers diglycidyliques de formule IX selon la revendication 1 dans lesquels R représente un radical répondant à la formule X:

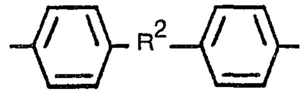

(X)

dans laquelle $R^2$ représente un radical méthylène ou isopropylène, et les symboles $R^1$ représentent chacun un alkyle contenant de 1 à 12 atomes de carbone.

5. Ethers diglycidylique selon la revendication 1 pris dans l'ensemble constitué par:
le bis-(p-(butoxy-3 glycidyloxy-2 propyloxy)-phényl)-2,2-propane
le bis-(p-(méthoxy-3 glycidyloxy-2 propyloxy)-phényl)-2,2-propane
le bis-(p-(éthoxy-3 glycidyloxy-2 propyloxy)-phényl)-2,2-propane
le bis-(p-(dodécyloxy-3 glycidyloxy-2 propyloxy)-phényl)-2,2-propane
le bis-(p-(tétradécyloxy-3 glycidyloxy-2 propyloxy)-phényl)-2,2-propane
le bis-(p-(benzyloxy-3 glycidyloxy-2 propyloxy)-phényl)-2,2-propane
le bis-(p-(butoxy-3 glycidyloxy-2 propyloxy)-phényl)-2,2-méthane
le bis-(phénoxy-3 glycidyloxy-2 propyloxy)-1,3 benzène,
le bis-(p-(butoxy-3 glycidyloxy-2 propyloxy)-phényl)-2,2-sulfone,
le bis-(p-(cyclohexyloxy-3 glycidyloxy-2 propyloxy)-phényl)-2,2-propane
le bis-(p-((butoxy-3 glycidyloxy-2 propyloxy)-4 dibromo-3,5-phényl)-2,2-propane.
le bis-(p-(allyloxy-3 glycidyloxy-2 propyloxy)-phényl)-2,2-propane et
le bis-(p-(phénoxy-3 glycidyloxy-2 propyloxy)-phényl)-2,2-propane.

6. Procédé de préparation d'éthers diglycidyliques de formule IX selon la revendication 1, procédé caractérisé en ce qu'on fait réagir un alcool di-secondaire, répondant à la formule XI:

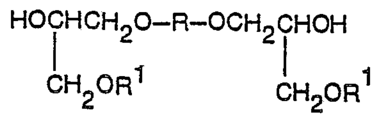

(XI)

dans laquelle
R et $R^1$ ont les mêmes significations que dans la formule IX, avec l'épichlorhydrine ou la dichlorhydrine en 1,3 du glycérol, réaction qui conduit à une bis-(chlorhydrine) répondant à la formula XII:

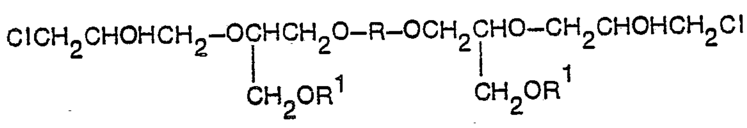

(XII)

dans laquelle
R et $R^1$ ont les mêmes significations que dans la formule IX, puis on déshydrochlore la bis-(chlorhydrine) de formule XII de manière connue.

7. Procédé selon la revendication 6 caractérisé en ce qu'on fait réagir l'alcool de formule XI avec au moins 1,5 équivalent d'épichlorhydrine ou de dichlorhydrine en 1,3 du glycérol, en présence d'un acide de Lewis comme catalyseur, puis on triate la bis-(chlorhydrine) de formule XII correspondante par un agent alcalin.

8. Procédé selon la revendication 6 caractérisé en ce que le traitement de l'alcool de formule XI par l'épichlorhydrine ou la dichlorhydrine en 1,3 du glycérol est effectué dans un intervalle de température de 40 à 100°C.

9. Procédé selon la revendication 6 caractérisé en ce qu'on utilise un alcool de formule XI que l'on a préparé en faisant réagir:

a) de 1,5 à 2,5 mol d'un éther monoglycidylique de formule XIII:

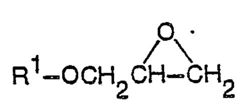

(XIII)

avec 1 mol d'un diphénol de formule XIV:

$$HO—R—OH \quad (XIV)$$

b) au moins 2 mol d'un alcool de formule XV:

$$R^1—OH \quad (XV)$$

avec 1 mol d'un éther diglycidylique de diphénol répondant à la formule XVI:

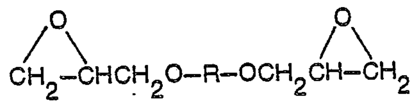

(XVI),

ou

c) 1 mol d'un sel de métal alcalin d'un diphénol de formule XIV avec environ 2 mol d'une chlorhydrine de formule XVII:

$$R^1—OCH_2CHOHCH_2Cl \qquad (XVII)$$

les symboles

R et $R^1$ présents dans ces diverses formules ayant les mêmes significations que dans la revendication 6.

10. Mélanges durcissables qui contiennent un éther diglycidylique selon la revendication 1 et un durcisseur pour résines époxydiques.